Europäisches Patentamt

European Patent Office

Office européen des brevets

(11) Veröffentlichungsnummer: **0 314 023 B1**

(12) **EUROPÄISCHE PATENTSCHRIFT**

(45) Veröffentlichungstag der Patentschrift: **11.05.94**

(21) Anmeldenummer: **88117624.2**

(22) Anmeldetag: **22.10.88**

(51) Int. Cl.⁵: **C07K 7/06**, G01N 33/86

(54) Glutaminhaltige Peptide, Verfahren zu deren Herstellung und Verwendung.

(30) Priorität: **29.10.87 DE 3736589**

(43) Veröffentlichungstag der Anmeldung:
**03.05.89 Patentblatt 89/18**

(45) Bekanntmachung des Hinweises auf die
Patenterteilung:
**11.05.94 Patentblatt 94/19**

(84) Benannte Vertragsstaaten:
**AT BE CH DE ES FR GB IT LI LU NL SE**

(56) Entgegenhaltungen:
**EP-A- 0 155 199**
**EP-A- 0 238 473**

**CHEMICAL ABSTRACTS, Band 100, 1984, Columbus, OH (US); J.J. GORMAN et al., Seite 619, Nr. 192243j&NUM;**

(73) Patentinhaber: **BEHRINGWERKE Aktiengesellschaft**
**Postfach 1140**
**D-35001 Marburg(DE)**

(72) Erfinder: **Stüber, Werner, Dr.**
**Cölber Weg 12**
**D-3551 Lahntal(DE)**

EP 0 314 023 B1

**Beschreibung**

Gegenstand der Erfindung sind Peptide der Struktur

H-$A_1$-$A_2$-$A_3$-$A_4$-Gln-$A_6$-Lys-Val-$A_9$-$A_{10}$-NH$_2$     (I)

worin

| | |
|---|---|
| $A_1$ | = Val, Leu |
| $A_2$ | = Gly, Ser |
| $A_3$ | = Pro, Hyp |
| $A_4$ | = Gly, Ser |
| $A_6$ | = Gly, Ser |
| $A_9$ | = Leu, Ile und |
| $A_{10}$ | = Gly, Ala sind, |

und ihre Salze, ein Verfahren zu deren Herstellung und deren Verwendung. Diese Peptide wirken als Substrat des Blutgerinnungsfaktors XIII und können zur Quantifizierung dieses Enzyms sowie zum Nachweis von Reaktionen dienen, in denen der aktivierte Blutgerinnungsfaktor XIII entsteht, verbraucht oder inhibiert wird.

Die vorliegende Erfindung gründet sich auf der Wirkungsweise der Transglutaminase F XIII, die in verschiedenen physiologischen Prozessen den Einbau von primären Aminen in Proteine bewirkt, die die Aminosäure Glutamin als Amin-Akzeptor besitzen. Bei dieser Reaktion, die im Rahmen der Blutgerinnung die Entstehung eines unlöslichen Blutgerinnsels bewirkt, wird als Nebenprodukt Ammoniak gebildet. Es gibt Methoden, die es gestatten, aus einer nachgeschalteten Ammoniakmessung die F XIII-Konzentration einer Probe zu ermitteln. Beispielsweise kann der Ammoniak, der durch den F XIII-vermittelten Einbau von Ethylamin in acetyliertes $\beta$-Kasein kontinuierlich entsteht, durch eine NADH-REaktion gemessen werden. Bei dieser NADH-Reaktion wird der entstehende Ammoniak unter der Wirkung des Enzyms GLDH in alpha-Ketoglutarat eingebaut. Hierbei entsteht Glutaminsäure and gleichzeitig wird NADH unter der Bildung von NAD$^+$ verbraucht. Da sich NADH und NAD$^+$ im spektralen Verhalten deutlich unterscheiden, ergibt eine Messung der Extinktionsabnahme, beispielsweise bei 340 nm, die sich ändernde Ammoniakkonzentration, und aus dieser Kinetik wird die F XIII-Konzentration ermittelt.

Der Nachteil dieser Methode liegt darin, daß als Substrat Kasein und zwar $\beta$-Kasein verwendet wird, das dephosphoryliert und N-acetyliert werden muß. Da nur ein Glutamin in der Proteinstruktur zum Amineinbau benutzt wird, werden verhältnismäßig hohe Substratkonzentrationen benötigt. In Clin.Chem 31/12, 2044-2045, 1985 ist beschrieben, daß der Einsatz von bestimmten glutaminhaltigen Peptiden diese Nachteile verbessert, da dem F XIII eindeutig definierte Substrate angeboten werden. Die Eignung solcher Peptidsubstrate kann aus dem kinetischen Verhalten, das heißt aus der Änderungsgeschwindigkeit der optischen Dichte (Delta OD/Zeit) ermittelt werden, wobei natürlicherweise hohe Werte günstig sind, um auch niedere Konzentrationen von F XIII noch hinreichend genau bestimmen zu können.

Aufgabe dieser Erfindung war es, glutaminhaltige Peptide mit gegenüber dem genannten Stand der Technik überlegenen Eigenschaften, das heißt hohen Umsatzraten und damit hohen Delta OD/Zeit-Werten zu schaffen.

Überraschenderweise wurde nun gefunden, daß Peptide der oben angegebenen Struktur I diese Bedingung in besonders geeigneter Weise erfüllen. Ein wesentlicher Unterschied zum Stand der Technik und damit die Neuheit ist durch die Anwesenheit von Prolin oder Hydroxyprolin in der 3-Stellung gegeben.

Gegenstand der Erfindung sind daher Peptide der oben in der Formel I mit den zugehörigen Definitionen angegebenen Struktur.

Die zum Aufbau der Peptide eingesetzten Aminosäuren können sowohl in der D- als auch L-Form vorliegen, wobei jedoch die L-Form bevorzugt wird. Günstig erweist sich der Einbau einer D-Aminosäure am N-Terminus, da hierdurch ein unerwünschter enzymatischer Abbau des Substrates verhindert werden kann.

Im besonderen eignen sich die folgenden Peptide (sofern nicht weiter vermerkt, liegen allgemein die Aminosäuren in der L Form vor):

EP 0 314 023 B1

```
H-Leu-Gly-Pro-Gly-Gln-Gly-Lys-Val-Leu-Gly-NH2
H-Leu-Gly-Hyp-Gly-Gln-Gly-Lys-Val-Leu-Gly-NH2
H-Leu-Gly-Pro-Gly-Gln-Ser-Lys-Val-Leu-Gly-NH2
H-Leu-Gly-Hyp-Gly-Gln-Ser-Lys-Val-Leu-Gly-NH2
H-Leu-Ser-Pro-Ser-Gln-Ser-Lys-Val-Leu-Gly-NH2
H-Leu-Ser-Hyp-Ser-Gln-Ser-Lys-Val-Leu-Gly-NH2
H-Leu-Gly-Pro-Gly-Gln-Ser-Lys-Val-Ile-Gly-NH2
H-D-Leu-Gly-Pro-Gly-Gln-Ser-Lys-Val-Ile-Gly-NH2
H-D-Leu-Gly-Pro-Gly-Gln-Ser-Lys-Val-Leu-Gly-NH2
```

Die erfindungsgemäßen Peptide können dabei in Form ihrer Salze vorliegen, beispielsweise als Chloride, Acetate, Formiate, Bromide oder Methansulfonate.

Die Herstellung dieser Peptide kann nach an sich bekannten Verfahren erfolgen, beispielsweise nach der klassischen in Lösung arbeitenden Technik, wobei einzelne geschützte Aminosäuren oder auch Peptidsegmente aneinander kondensiert werden und nach Abspaltung der Schutzgruppen das gewünschte Peptid erhalten wird. Als Schutzgruppen der alpha-Aminogruppe dienen hierzu beispielsweise Boc, Z, Ddz, Bpoc oder Fmoc (Abkürzungen: siehe weiter unten). Als Seitenschutzgruppen des Lysins benutzt man Boc, Z, o-ClZ, oBrZ oder TFA und für Serin Bzl oder t-Bu.

Vorzugsweise eignet sich für die Herstellung der erfindungsgemäßen Peptide der Einsatz von Peptidsegmenten, die dann zu den endgültigen Sequenzen aufgebaut werden. Als günstig hat es sich erwiesen, das Peptidsegment $A_1$-$A_2$-$A_3$-$A_4$ an das Segment Gln-$A_6$-Lys-Val-$A_9$-$A_{10}$-NH2 zu koppeln. Die Aminogruppe von $A_1$ muß mit einer Schutzgruppe versehen werden, wobei entsprechend der obigen Ausführung Boc oder Z ganz besonders bevorzugt ist. Ist $A_3$ Hydroxyprolin, so erweist sich die t-Bu-Gruppe an der Hydroxylgruppe als vorteilhaft. Die epsilon-Aminogruppe des C-terminalen Hexapeptids wird ebenfalls geschützt. Der Aufbau der einzelnen hier genannten Peptidsegmente erfolgt unter Einsatz einzelner geschützter Aminosäuren in Lösung, wobei sich Lösemittel, wie Dimethylformamid, N-Methylpyrrolidon und Dimethylsulfoxid, bevorzugt eignen. Die Aktivierungsschritte der Carboxylgruppe werden vorzugsweise mit einem Carbodiimid, besonders bevorzugt Dicylohexylcarbodiimid, in Gegenwart von Hydroxybenzotriazol oder Hydroxysuccinimid ausgeführt. Die pH-Werte bei den Kopplungsschritten werden im Bereich von 4 bis 10 gehalten, wobei eine Base, wie N-Methylmorpholin, bevorzugt benutzt wird, so daß sich ein pH-Wert von 6 bis 8 ergibt. Die einzelnen Peptidsegmente, wie auch die geschützten Dekapeptide werden nach bekannten Methoden gereinigt. Vorzugsweise werden die Peptide in organischen Lösemitteln, die bekanntlich als Lösemittel für Peptide geeignet sind, beispielsweise Ethylacetat oder Butanol, und nicht vollständig mit Wasser mischbar sind, gelöst und die Peptidlösungen mit Wasser, 1 M Kaliumhydrogensulfat und 1 M Natriumhydrogensulfat gewaschen. Die erfindungsgemäßen Peptide, entsprechend Formel (I), werden vorzugsweise durch Gelpermeationschromatographie, bevorzugt an [R]Sephadex-G25 unter Verwendung von 1%iger Essigsäure (Vol.) gereinigt.

Darüberhinaus eignet sich für die Herstellung der erfindungsgemäßen Peptide die Festphasenmethode. Dabei werden die Peptide an einer Matrix wie quervernetztem Polystyrol, Polyacrylamid oder dergleichen, die mit entsprechenden Ankern versehen sind, aufgebaut und davon selektiv abgespalten.

Gegenstand der Erfindung ist deshalb auch ein Verfahren zur Herstellung der erfindungsgemäßen Peptide mittels der Festphasen-Technik zur Peptidsynthese. Entsprechend dem Stand der Technik müssen die Festphasen mit sogenannten Ankermolekülen versehen sein, die eine Abspaltung der Peptide in Form ihrer Amidderivate gestatten. Solche Ankerverbindungen sind beispielsweise immobilisierte Benzhydrylaminderivate, an die dann die in der Sequenz gekoppelt wird. Als Lösemittel wird hierzu vorzugsweise Dichlormethan, N-Methylpyrrolidon und ganz besonders bevorzugt Dimethylformamid benutzt. Die Aktivierung der Aminosäuren erfolgt beispielsweise über Aktivester, gemischte oder symmetrische Anhydride oder Carbodiimidaktivierungen. Die Abspaltung der Peptide vom Träger erfolgt vorzugsweise acidolytisch wobei gleichzeitig die Seitenschutzgruppen entfernt werden. Die Peptide werden dann nach an sich bekannter Methode gereinigt, besonders bevorzugt durch Gelpermeationschromatographie.

Bevorzugt ist für die Festphasenpeptidsynthese die Verwendung von quervernetztem, aminofunktionalisiertem Polystyrol (1 g Vernetzer auf 100 g Polystyrol). Die C-terminale Aminosäure wird besonders

3

bevorzugt über Benzhydrylaminderivate an den polymeren Träger gekoppelt, wobei ganz besonders bevorzugt Fmoc-Aminosäure-(4-carboxylatomethyloxyphenyl-4'-methoxyphenyl)methylamid an Aminogruppen der Matrix über eine Carbodiimid vermittelte Reaktion gebunden werden.

Nach Abspalten der Fmoc-Gruppe und DMF-Isopropanol-Waschschritten wird die in der Sequenz folgende geschützte Aminosäure in 3fach molarem Überschuß bezogen auf die Menge an der Festphase nachweisbaren Aminogruppen sowie der 4,5fache Überschuß an HOBt zugesetzt. Nach Zugabe eines 3,3fachen Überschusses (bezüglich der Aminogruppen) an Diisopropylcarbodiimid oder Dicyclohexylcarbodiimid wird die Kopplung während einer Stunde ausgeführt. Daraufhin werden überschüssige Reagenzien durch Waschen mit DMF und Isopropanol entfernt.

Die erfindungsgemäßen Peptide werden durch eine acidolytische Behandlung, bevorzugt mit einem Gemisch von 4 Volumteilen Trifluoressigsäure und einem Volumteil eines Gemischs aus Methylphenylsulfid/Dimercaptoethan 3:1 (v:v), von der Festphase abgespalten. Die Rohpeptide werden durch Zusatz von Ether, bevorzugt Diethylether, gefällt und durch übliche, an sich bekannte Reinigungsschritte, gereinigt. Bevorzugt werden die Ionenaustauschchromatographie oder die Gelpermation, beispielsweise an $^R$Sephadex G-25 mit 0,5 ml/100 ml Essigsäure als Eluent.

Zur Prüfung der Peptide auf ihre Eignung als Substrate für die Transglutaminase F XIII werden eine Faktor XIII enthaltende Probelösung in einem Puffergemisch, das etwa 30 mmol Calciumchlorid enthielt, bei pH 7,6 ± 0,5 mit Thrombin aktiviert. Es werden nun die Peptidlösungen, eine Lösung eines Aminderivates, beispielsweise Glycinethylester oder Glycinmethylester, Ketoglutaratpuffer, Glutamindehydrogenase und eine NADH-Lösung zugesetzt. Damit wird die Freisetzung von Ammoniak aus dem Dekapeptidamid anhand der Extinktionsabnahme bei 340 ± 15 nm gemessen und die Aktivität an F XIII bestimmt.

Überraschenderweise wurde festgestellt, daß sich die erfindungsgemäßen Peptide sehr gut zum Nachweis des F XIII eignen. Wichtig ist, daß diese Peptide als $A_3$ in der Formel Prolin oder eine verwandte Aminosäure, beispielsweise Hydroxyprolin, aufweisen. Im Rahmen von vergleichenden Untersuchungen konnte überraschend gezeigt werden, daß die Spaltgeschwindigkeit, gemessen als Änderung der Extinktion pro Zeiteinheit, gegenüber bekannten Substanzen größer ist.

Mit diesen Substraten gelingt es deshalb, die Nachweisgrenze für F XIII herabzusetzen und höhere Genauigkeiten bei der Messung zu erzielen. Gegenstand der Erfindung ist deshalb auch die Verwendung von Peptiden der Formel I in einem Verfahren zur Bestimmung von Glutaminasen, vorzugsweise F XIII.

Die folgenden Beispiele beschreiben die Erfindung näher:

Beispiel 1

Synthese von H-Leu-Gly-Pro-Gly-Gln-Gly-Lys-Val-Leu-Gly-NH$_2$

1 g Aminomethylpolystryrol (1 g/100 g Vernetzer; 0,49 mmol NH$_2$-Gruppen/g) wurden in 15 ml DMF gequollen und in einem Peptidsynthesizer SP 640 von $^R$Labortec AG zur Reaktion gebracht. Beginnend mit der C-terminalen Aminosäure wurde nach Ablauf der üblichen Harzwaschschritte 1,5 m Mol Fmoc-Glyin-(4-carboxylatomethyloxyphenyl-4'-methoxyphenyl)methylamid und 2,25 m Mol HOBt in 15 ml DMF gelöst, zum Harz zugesetzt und mit 1,65 m Mol Diisopropylcarbodiimid aktiviert. Der Reaktionsansatz wurde eine Stunde bei Raumtemperatur geschüttelt, daraufhin wurde das Polymer durch Waschschritte mit DMF und Isopropanol von überschüssigen Reagenzien und Nebenprodukten befreit. Die Fmoc-Schutzgruppe wurde durch 10 minütige Reaktion mit einer 20 ml/100 ml Piperidinlösung in DMF abgespalten.

Dieser Reaktionszyklus wurde bis zur N-terminalen Aminosäure beibehalten. Es wurden die folgenden Aminosäurederivate eingesetzt: Fmoc-Leu, Fmoc-Val, Fmoc-Lys (Boc), Fmoc-Gly, Fmoc-Gln, Fmoc-Pro, Boc-Leu (N-terminale Aminosäure).

Das Peptidpolymer wurde mit 16 ml TFA, 3 ml Thioanisol und 1 ml Ethandithiol 2 Stunden bei 35 °C behandelt. Die peptidhaltige Lösung wurde nach Filtration mit Diethylether versetzt und das kristallisierte Peptid abfiltriert und getrocknet. Das Rohpeptid wurde in 20 ml 0,5 ml/100 ml Essigsäure gelöst und in einer $^R$Sephadex G-25 Säule gereinigt. Die Peptidfraktion wurde lyophilisiert. Ausbeute: 370 mg

Beispiel 2

Synthese von H-Leu-Ser-Hyp-Ser-Gln-Ser-Lys-Val-Leu-Gly-NH$_2$

In Analogie zu Beispiel 1 wurde dieses Peptid mit denselben Aminosäurederivaten aufgebaut, ausgenommen Fmoc-Ser (tBu) und Fmoc-Hyp (tBu). Die Arbeitsschritte und Mengenangaben entsprechen Beispiel 1.

Die anderen Peptide werden analog synthetisiert.

Beispiel 3

Bestimmung der Faktor XIII-Aktivität durch photometrische Messung

50 $\mu$l einer Faktor XIII (0,6 E)-enthaltenden Lösung, die auf pH 7,6 eingestellt wurde, und 50 mmolar HEPES, 150 mmolar Natriumchlorid und 30 mmolar $CaCl_2$ enthielt, wurde mit 25 $\mu$l Thrombinlösung (30 Einheiten gelöst in 1 ml physiologischer Kochsalzlösung) 10 min bei 37°C in einer 1 ml Küvette inkubiert. Es wurden 700 $\mu$l einer Pufferlösung, bestehend aus Triethanolamin pH 8,0, 2,2 g alpha-Ketoglutarat/Liter und 133 mg NADH/Liter zugesetzt. Die Probelösung wurde daraufhin mit 25 $\mu$l Lösung von 5 ml Glutamatdehydrogenase (etwa 120 Einheiten /mg), 100 ml Glycerin, pH 7, 100$\mu$l Glycinethylester (30 mg/ml in $H_2O$) und 100 $\mu$l Dekapeptidamidlösung (10 mg/ml in $H_2O$) versetzt. Es wurde die Extinktionsabnahme gemessen. Die Ergebnisse der kinetischen Messung bei 340 nm sind in Form von Delta OD/min-Werten aus der folgenden Tabelle ersichtlich.

| <u>Peptidsequenz</u> | <u>Delta OD/min</u> |
|---|---|
| Leu-Ser-Leu-Ser-Gln-<br>Ser-Lys-Val-Leu-Gly-NH$_2$ | 0.21 |
| Leu-Gly-Gly-Gly-Gln-<br>Gly-Lys-Val-Leu-Gly-NH$_2$ | 0.12 |
| Leu-Gly-Pro-Gly-Gln-<br>Ser-Lys-Val-Leu-Gly-NH$_2$ | 0.32 |
| Leu-Gly-Hyp-Gly-Gln-<br>Ser-Lys-Val-Leu-Gly-NH$_2$ | 0.27 |
| Leu-Gly-Pro-Gly-Gln-<br>Ser-Lys-Val-Ile-Gly-NH$_2$ | 0.35 |

Abkürzungen

| NADH | Nicotinamid-dinucleotid, reduziert |
|---|---|
| NAD$^+$ | Nicotinamid-dinucleotid |
| GLDH | Glutamiatdehydrogenase |
| nm | Nanometer |
| Val | Valin |
| Leu | Leucin |
| Gly | Glycin |
| Ser | Serin |
| Pro | Prolin |
| Hyp | Hydroxyprolin |
| Ile | Isoleucin |
| Ala | Alanin |

| | |
|---|---|
| Gln | Glutamin |
| Lys | Lysin |
| Boc | tert.-Butyloxycarbonyl |
| Z | Benzyloxycarbonyl |
| o-ClZ | o-Chlorbenzyloxycarbonyl |
| o-BrZ | o-Brombenzyloxycarbonyl |
| TFA | Trifluoressigsäure bzw. Trifluoracetyl |
| Bzl | Benzyl |
| t-Bu | tert.-Butyl |
| HOBt | Hydroxybenzotriazol |
| DMF | Dimethylformamid |
| OD | Optische Dichte |

**Patentansprüche**

**Patentansprüche für folgende Vertragsstaaten : AT, BE, CH, DE, FR, GB, IT, LI, LU, NL, SE**

1. Peptide der Struktur

$$H-A_1-A_2-A_3-A_4-Gln-A_6-Lys-Val-A_9-A_{10}-NH_2 \qquad (I)$$

worin
$A_1$ = Val, Leu
$A_2$ = Gly, Ser
$A_3$ = Pro, Hyp
$A_4$ = Gly, Ser
$A_6$ = Gly, Ser
$A_9$ = Leu, Ile und
$A_{10}$ = Gly, Ala sind,
und ihre Salze.

2. Peptide nach Anspruch 1, dadurch gekennzeichnet, daß die Aminosäuren in der L-Form vorliegen.

3. Peptide nach Anspruch 1, dadurch gekennzeichnet, daß $A_1$ in der D-Form vorliegt.

4. Peptide nach Anspruch 1 mit der Formel

$$H-Leu-Gly-Pro-Gly-Gln-Gly-Lys-Val-Leu-Gly-NH_2$$
$$H-Leu-Gly-Hyp-Gly-Gln-Gly-Lys-Val-Leu-Gly-NH_2$$
$$H-Leu-Gly-Pro-Gly-Gln-Ser-Lys-Val-Leu-Gly-NH_2$$
$$H-Leu-Gly-Hyp-Gly-Gln-Ser-Lys-Val-Leu-Gly-NH_2$$
$$H-Leu-Ser-Pro-Ser-Gln-Ser-Lys-Val-Leu-Gly-NH_2$$
$$H-Leu-Ser-Hyp-Ser-Gln-Ser-Lys-Val-Leu-Gly-NH_2$$
$$H-Leu-Gly-Pro-Gly-Gln-Ser-Lys-Val-Ile-Gly-NH_2$$
$$H-D-Leu-Gly-Pro-Gly-Gln-Ser-Lys-Val-Ile-Gly-NH_2 \quad oder$$
$$H-D-Leu-Gly-Pro-Gly-Gln-Ser-Lys-Val-Leu-Gly-NH_2 .$$

5. Verfahren zur Herstellung eines Peptids der Struktur

$$H-A_1-A_2-A_3-A_4-Gln-A_6-Lys-Val-A_9-A_{10}-NH_2 \qquad (I)$$

worin

$A_1$ = Val, Leu
$A_2$ = Gly, Ser
$A_3$ = Pro, Hyp
$A_4$ = Gly, Ser
$A_6$ = Gly, Ser
$A_9$ = Leu, Ile und
$A_{10}$ = Gly, Ala sind,

und gegebenenfalls eines ihrer Salze, dadurch gekennzeichnet, daß geschützte Aminosäurederivate oder Peptidsegmente in Lösung oder an einer Festphase aneinander gekoppelt werden und durch Abspaltung der Schutzgruppen sowie im Falle einer Festphase durch Abspaltung vom Trägerharz Peptide gemäß Struktur (I) erhalten werden.

6.   Verwendung eines Peptids der Formel I in Anspruch 1 in einem "in vitro" Verfahren zur Quantifizierung des Blutgerinnungsfaktors XIII.

7.   Verwendung eines Peptids der Formel I in Anspruch 1 in einem "in vitro" Verfahren zum Nachweis von Reaktionen, in denen der aktivierte Blutgerinnungsfaktor XIII entsteht, verbraucht oder inhibiert wird.

**Patentansprüche für folgenden Vertragsstaat : ES**

1.   Verfahren zur Herstellung eines Peptids der Struktur

$H-A_1-A_2-A_3-A_4-Gln-A_6-Lys-Val-A_9-A_{10}-NH_2$   (I)

worin
$A_1$ = Val, Leu
$A_2$ = Gly, Ser
$A_3$ = Pro, Hyp
$A_4$ = Gly, Ser
$A_6$ = Gly, Ser
$A_9$ = Leu, Ile und
$A_{10}$ = Gly, Ala sind,

und gegebenenfalls eines seiner Salze, dadurch gekennzeichnet, daß geschützte Aminosäurederivate oder Peptidsegmente in Lösung oder an einer Festphase aneinander gekoppelt werden und durch Abspaltung der Schutzgruppen sowie im Falle einer Festphase durch Abspaltung vom Trägerharz Peptide gemäß Struktur (I) erhalten werden.

2.   Verfahren nach Anspruch 1, dadurch gekennzeichnet, daß ein Peptid der Struktur

$H-A_1-A_2-A_3-A_4-Gln-A_6-Lys-Val-A_9-A_{10}-NH_2$   (I)

worin
$A_1$ = Val, Leu
$A_2$ = Gly, Ser
$A_3$ = Pro, Hyp
$A_4$ = Gly, Ser
$A_6$ = Gly, Ser
$A_9$ = Leu, Ile und
$A_{10}$ = Gly, Ala sind,

oder eines seiner Salze hergestellt wird.

3.   Verfahren nach Anspruch 1, dadurch gekennzeichnet, daß die Aminosäuren in der L-Form vorliegen.

4.   Verfahren nach Anspruch 1, dadurch gekennzeichnet, daß $A_1$ in der D-Form vorliegt.

5. Verfahren nach Anspruch 1, wobei ein Peptid mit der Formel

```
H-Leu-Gly-Pro-Gly-Gln-Gly-Lys-Val-Leu-Gly-NH2
H-Leu-Gly-Hyp-Gly-Gln-Gly-Lys-Val-Leu-Gly-NH2
H-Leu-Gly-Pro-Gly-Gln-Ser-Lys-Val-Leu-Gly-NH2
H-Leu-Gly-Hyp-Gly-Gln-Ser-Lys-Val-Leu-Gly-NH2
H-Leu-Ser-Pro-Ser-Gln-Ser-Lys-Val-Leu-Gly-NH2
H-Leu-Ser-Hyp-Ser-Gln-Ser-Lys-Val-Leu-Gly-NH2
H-Leu-Gly-Pro-Gly-Gln-Ser-Lys-Val-Ile-Gly-NH2
H-D-Leu-Gly-Pro-Gly-Gln-Ser-Lys-Val-Ile-Gly-NH2 oder
H-D-Leu-Gly-Pro-Gly-Gln-Ser-Lys-Val-Leu-Gly-NH2
```

hergestellt wird.

**Claims**
**Claims for the following Contracting States : AT, BE, CH, DE, FR, GB, IT, LI, LU, NL, SE**

1. A peptide of the structure

$$\text{H-}A_1\text{-}A_2\text{-}A_3\text{-}A_4\text{-Gln-}A_6\text{-Lys-Val-}A_9\text{-}A_{10}\text{-NH}_2 \qquad (I)$$

in which

| | |
|---|---|
| $A_1$ | = Val, Leu |
| $A_2$ | = Gly, Ser |
| $A_3$ | = Pro, Hyp |
| $A_4$ | = Gly, Ser |
| $A_6$ | = Gly, Ser |
| $A_9$ | = Leu, Ile and |
| $A_{10}$ | = Gly, Ala, |

and the salts thereof.

2. A peptide as claimed in claim 1, wherein the amino acids are in the L form.

3. A peptide as claimed in claim 1, wherein $A_1$ is in the D form.

4. A peptide as claimed in claim 1 having the formula

```
H-Leu-Gly-Pro-Gly-Gln-Gly-Lys-Val-Leu-Gly-NH2
H-Leu-Gly-Hyp-Gly-Gln-Gly-Lys-Val-Leu-Gly-NH2
H-Leu-Gly-Pro-Gly-Gln-Ser-Lys-Val-Leu-Gly-NH2
H-Leu-Gly-Hyp-Gly-Gln-Ser-Lys-Val-Leu-Gly-NH2
H-Leu-Ser-Pro-Ser-Gln-Ser-Lys-Val-Leu-Gly-NH2
H-Leu-Ser-Hyp-Ser-Gln-Ser-Lys-Val-Leu-Gly-NH2
H-Leu-Gly-Pro-Gly-Gln-Ser-Lys-Val-Ile-Gly-NH2
```

8

EP 0 314 023 B1

**H-D-Leu-Gly-Pro-Gly-Gln-Ser-Lys-Val-Ile-Gly-NH$_2$ or**

**H-D-Leu-Gly-Pro-Gly-Gln-Ser-Lys-Val-Leu-Gly-NH$_2$.**

5. A process for the preparation of a peptide of the structure

$\text{H-A}_1\text{-A}_2\text{-A}_3\text{-A}_4\text{-Gln-A}_6\text{-Lys-Val-A}_9\text{-A}_{10}\text{-NH}_2$ (I)

in which

| | |
|---|---|
| A$_1$ | = Val, Leu |
| A$_2$ | = Gly, Ser |
| A$_3$ | = Pro, Hyp |
| A$_4$ | = Gly, Ser |
| A$_6$ | = Gly, Ser |
| A$_9$ | = Leu, Ile and |
| A$_{10}$ | = Gly, Ala |

and, where appropriate, one of its salts, which comprises coupling together protected amino acid derivatives or peptide segments in solution or on a solid phase, and obtaining a peptide of the structure (I) by elimination of the protective groups and, in the case of a solid phase, by elimination from the support resin.

6. The use of a peptide of the formula I in claim 1 in an in vitro method for the quantification of blood coagulation factor XIII.

7. The use of a peptide of the formula I in claim 1 in an in vitro method for detecting reactions in which activated blood coagulation factor XIII is produced, consumed or inhibited.

**Claims for the following Contracting State : ES**

1. A process for the preparation of a peptide of the structure

$\text{H-A}_1\text{-A}_2\text{-A}_3\text{-A}_4\text{-Gln-A}_6\text{-Lys-Val-A}_9\text{-A}_{10}\text{-NH}_2$ (I)

in which

| | |
|---|---|
| A$_1$ | = Val, Leu |
| A$_2$ | = Gly, Ser |
| A$_3$ | = Pro, Hyp |
| A$_4$ | = Gly, Ser |
| A$_6$ | = Gly, Ser |
| A$_9$ | = Leu, Ile and |
| A$_{10}$ | = Gly, Ala |

and, where appropriate, one of its salts, which comprises coupling together protected amino acid derivatives or peptide segments in solution or on a solid phase, and obtaining a peptide of the structure (I) by elimination of the protective groups and, in the case of a solid phase, by elimination from the support resin.

2. The process as claimed in claim 1, which comprises preparing a peptide of the structure

$\text{H-A}_1\text{-A}_2\text{-A}_3\text{-A}_4\text{-Gln-A}_6\text{-Lys-Val-A}_9\text{-A}_{10}\text{-NH}_2$ (I)

in which

| | |
|---|---|
| A$_1$ | = Val, Leu |
| A$_2$ | = Gly, Ser |
| A$_3$ | = Pro, Hyp |
| A$_4$ | = Gly, Ser |
| A$_6$ | = Gly, Ser |

9

$A_9$ = Leu, Ile and
$A_{10}$ = Gly, Ala,
or one of its salts.

3. The process as claimed in claim 1, wherein the amino acids are in the L form.

4. The process as claimed in claim 1, wherein $A_1$ is in the D form.

5. The process as claimed in claim 1, in which a peptide having the formula

```
H-Leu-Gly-Pro-Gly-Gln-Gly-Lys-Val-Leu-Gly-NH₂
H-Leu-Gly-Hyp-Gly-Gln-Gly-Lys-Val-Leu-Gly-NH₂
H-Leu-Gly-Pro-Gly-Gln-Ser-Lys-Val-Leu-Gly-NH₂
H-Leu-Gly-Hyp-Gly-Gln-Ser-Lys-Val-Leu-Gly-NH₂
H-Leu-Ser-Pro-Ser-Gln-Ser-Lys-Val-Leu-Gly-NH₂
H-Leu-Ser-Hyp-Ser-Gln-Ser-Lys-Val-Leu-Gly-NH₂
H-Leu-Gly-Pro-Gly-Gln-Ser-Lys-Val-Ile-Gly-NH₂
H-D-Leu-Gly-Pro-Gly-Gln-Ser-Lys-Val-Ile-Gly-NH₂ or
H-D-Leu-Gly-Pro-Gly-Gln-Ser-Lys-Val-Leu-Gly-NH₂
```

is prepared.

**Revendications**

**Revendications pour les Etats contractants suivants : AT, BE, CH, DE, FR, GB, IT, LI, LU, NL, SE**

1. Peptides de structure

$H-A_1-A_2-A_3-A_4-Gln-A_6-Lys-Val-A_9-A_{10}-NH_2$     (I)

dans laquelle
$A_1$ = Val, Leu
$A_2$ = Gly, Ser
$A_3$ = Pro, Hyp
$A_4$ = Gly, Ser
$A_6$ = Gly, Ser
$A_9$ = Leu, Ile
$A_{10}$ = Gly, Ala,
et leurs sels.

2. Peptides selon la revendication 1, caractérisés en ce que les aminoacides se trouvent sous la forme L.

3. Peptides selon la revendication 1, caractérisés en ce que $A_1$ se trouve sous la forme D.

**4.** Peptides selon la revendication 1, de formule

```
H-Leu-Gly-Pro-Gly-Gln-Gly-Lys-Val-Leu-Gly-NH2
H-Leu-Gly-Hyp-Gly-Gln-Gly-Lys-Val-Leu-Gly-NH2
H-Leu-Gly-Pro-Gly-Gln-Ser-Lys-Val-Leu-Gly-NH2
H-Leu-Gly-Hyp-Gly-Gln-Ser-Lys-Val-Leu-Gly-NH2
H-Leu-Ser-Pro-Ser-Gln-Ser-Lys-Val-Leu-Gly-NH2
H-Leu-Ser-Hyp-Ser-Gln-Ser-Lys-Val-Leu-Gly-NH2
H-Leu-Gly-Pro-Gly-Gln-Ser-Lys-Val-Ile-Gly-NH2
H-D-Leu-Gly-Pro-Gly-Gln-Ser-Lys-Val-Ile-Gly-NH2 ou
H-D-Leu-Gly-Pro-Gly-Gln-Ser-Lys-Val-Leu-Gly-NH2.
```

**5.** Procédé pour la préparation d'un peptide de structure

$$H\text{-}A_1\text{-}A_2\text{-}A_3\text{-}A_4\text{-}Gln\text{-}A_6\text{-}Lys\text{-}Val\text{-}A_9\text{-}A_{10}\text{-}NH_2 \qquad (I)$$

dans laquelle

$A_1$ = Val, Leu
$A_2$ = Gly, Ser
$A_3$ = Pro, Hyp
$A_4$ = Gly, Ser
$A_6$ = Gly, Ser
$A_9$ = Leu, Ile
$A_{10}$ = Gly, Ala,

et éventuellement d'un de ses sels, caractérisé en ce qu'on assemble les uns avec les autres des dérivés protégés d'aminoacides ou des segments de peptides en solution ou sur une phase solide et on obtient des peptides de structure (I) par élimination des groupes protecteurs ainsi que, dans le cas d'une phase solide, par élimination de la résine de support.

**6.** Utilisation d'un peptide de formule I dans la revendication 1, dans un procédé in vitro pour la détermination quantitative du facteur de coagulation XIII.

**7.** Utilisation d'un peptide de formule I dans la revendication 1, dans un procédé in vitro pour la mise en évidence de réactions dans lesquelles le facteur de coagulation XIII activé est formé, consommé ou inhibé.

**Revendications pour l'Etat contractant suivant : ES**

**1.** Procédé pour la préparation d'un peptide de structure

$$H\text{-}A_1\text{-}A_2\text{-}A_3\text{-}A_4\text{-}Gln\text{-}A_6\text{-}Lys\text{-}Val\text{-}A_9\text{-}A_{10}\text{-}NH_2 \qquad (I)$$

dans laquelle

$A_1$ = Val, Leu
$A_2$ = Gly, Ser
$A_3$ = Pro, Hyp
$A_4$ = Gly, Ser
$A_6$ = Gly, Ser
$A_9$ = Leu, Ile
$A_{10}$ = Gly, Ala,

et éventuellement d'un de ses sels, caractérisé en ce qu'on couple les uns avec les autres des dérivés protégés d'aminoacides ou des segments de peptides en solution ou sur une phase solide et on obtient des peptides de structure (I) par élimination des groupes protecteurs ainsi que, dans le cas d'une phase solide, par élimination de la résine de support.

2. Procédé selon la revendication 1, caractérisé en ce qu'on prépare un peptide de structure

H-A$_1$-A$_2$-A$_3$-A$_4$-Gln-A$_6$-Lys-Val-A$_9$-A$_{10}$-NH$_2$      (I)

dans laquelle
A$_1$      = Val, Leu
A$_2$      = Gly, Ser
A$_3$      = Pro, Hyp
A$_4$      = Gly, Ser
A$_6$      = Gly, Ser
A$_9$      = Leu, Ile
A$_{10}$      = Gly, Ala,
ou l'un de ses sels.

3. Procédé selon la revendication 1, caractérisé en ce que les aminoacides se trouvent sous la forme L.

4. Procédé selon la revendication 1, caractérisé en ce que A$_1$ se trouve sous la forme D.

5. Procédé selon la revendication 1, dans lequel on prépare un peptide de formule

```
H-Leu-Gly-Pro-Gly-Gln-Gly-Lys-Val-Leu-Gly-NH₂
H-Leu-Gly-Hyp-Gly-Gln-Gly-Lys-Val-Leu-Gly-NH₂
H-Leu-Gly-Pro-Gly-Gln-Ser-Lys-Val-Leu-Gly-NH₂
H-Leu-Gly-Hyp-Gly-Gln-Ser-Lys-Val-Leu-Gly-NH₂
H-Leu-Ser-Pro-Ser-Gln-Ser-Lys-Val-Leu-Gly-NH₂
H-Leu-Ser-Hyp-Ser-Gln-Ser-Lys-Val-Leu-Gly-NH₂
H-Leu-Gly-Pro-Gly-Gln-Ser-Lys-Val-Ile-Gly-NH₂
H-D-Leu-Gly-Pro-Gly-Gln-Ser-Lys-Val-Ile-Gly-NH₂ ou
H-D-Leu-Gly-Pro-Gly-Gln-Ser-Lys-Val-Leu-Gly-NH₂.
```